(19) **Europäisches Patentamt**
**European Patent Office**
**Office européen des brevets**

(11) **EP 3 888 733 A1**

(12) **EUROPEAN PATENT APPLICATION**
published in accordance with Art. 153(4) EPC

(43) Date of publication:
06.10.2021 Bulletin 2021/40

(51) Int Cl.:
$A61M\ 25/10$ (2013.01)     $A61L\ 29/14$ (2006.01)
$A61L\ 29/16$ (2006.01)

(21) Application number: 19888788.7

(22) Date of filing: 27.11.2019

(86) International application number:
PCT/CN2019/121319

(87) International publication number:
WO 2020/108539 (04.06.2020 Gazette 2020/23)

(84) Designated Contracting States:
AL AT BE BG CH CY CZ DE DK EE ES FI FR GB GR HR HU IE IS IT LI LT LU LV MC MK MT NL NO PL PT RO RS SE SI SK SM TR
Designated Extension States:
BA ME
Designated Validation States:
KH MA MD TN

(30) Priority: 30.11.2018 CN 201811460227

(71) Applicant: Shanghai MicroPort Medical (Group) Co., Ltd.
Shanghai 201203 (CN)

(72) Inventors:
• DAI, Mingxin
Shanghai 201203 (CN)
• WU, Xiaolei
Shanghai 201203 (CN)
• GUO, Fang
Shanghai 201203 (CN)
• ZHAO, Yuegen
Shanghai 201203 (CN)
• QUE, Yiyun
Shanghai 201203 (CN)

(74) Representative: Patentship
Patentanwaltsgesellschaft mbH
Elsenheimerstraße 65
80687 München (DE)

(54) **MEDICAL TUBING AND PREPARATION METHOD THEREFOR**

(57) A porous balloon and a preparation method therefor; the porous balloon is used for drug loading, and the porous balloon is prepared by employing the following method: soaking an interventional carrier in an etching solution for etching so as to obtain a porous balloon (S3). The structure of the porous balloon is suitable for the storage and release of a drug, improves the binding force between a drug coating and a surface of the porous balloon, and solves the problems wherein a drug coating easily falls off a surface of a balloon during pushing and retraction, a drug is not sufficiently evenly covered on a surface, and the drug release rate and release speed are not high enough during release; in addition, the structure of the porous balloon does not reduce the physical properties of the balloon, avoiding the problem wherein etching grooves results in balloon properties, especially physical properties, being reduced, such as balloon rated burst pressure being reduced.

```
┌─────────────────────────────────────────────┐
│        Preparing an interventional carrier    │───⌇ S1
└─────────────────────────────────────────────┘
                      │
                      ▼
┌─────────────────────────────────────────────┐
│        Preparing an etching solution          │───⌇ S2
└─────────────────────────────────────────────┘
                      │
                      ▼
┌─────────────────────────────────────────────┐
│ Immersing the interventional carrier into the │
│ etching solution for etching to obtain the    │───⌇ S3
│ porous balloon                                │
└─────────────────────────────────────────────┘
```

Fig. 3

EP 3 888 733 A1

**Description**

<u>TECHNICAL FIELD</u>

The present application relates to the field of medical instruments, and in particular, to a porous balloon and preparation method thereof.

<u>BACKGROUND</u>

Interventional therapy is effective at treating coronary artery disease, peripheral artery disease, intracranial artery disease and other vascular diseases. The basic principle of interventional therapy is to deliver a carrier that is surface-coated with a particular drug to a target lesion site within a blood vessel via a guide catheter and a guide wire, and to mechanically dilate the carrier by pressurization, thereby bringing the drug coated on carrier surface into close contact with the focal blood vessel wall. As a result, the drug can be rapidly released and absorbed by the focal blood vessel wall so as to provide therapeutic effects on the blood vessel diseases.

The most important factor on the drug loading performance of a carrier used in interventional therapy is the coating of drugs on carrier surface. When reached the lesion site, the carrier is required to have good drug transfer properties to ensure rapid and uniform release of the drug into the blood vessel wall. However, during delivery of the carrier toward the lesion site and withdrawal of carrier after its dilation and release, the drug coating is required to keep a low loss rate, i.e., maintaining the drug on the carrier surface as much as possible to reduce fall-off of drug particles due to washing action of blood flow. Therefore, for the carrier, an increased drug loading performance is of particular importance.

Taking the drug-coated balloons (DCBs) for use in interventional therapy as an example, there are about 10 or more DCB products all over the world that have been, or are to be, approved for commercialization, such as the SeQuent Please balloons manufactured by B. Braun. Most of these DCB products are prepared by coating surfaces of the bare balloon with the drug paclitaxel and other mixed reagents. The drug loading performance of DCB may be improved by selections of various drugs and mixed reagents and the ratio between drugs and mixed reagents. However, such an approach of improving the drug coating itself fails to benefit DCBs' drug loading performance much because changing the composition of the drug and mixed reagents could not primely solve the problems including easy stripping off of the drug coating during the delivery and withdrawal processes, uneven coating of the drug over the balloon surface, and low drug release rate and low drug release speed during release.

In addition, there are also techniques for enhancing drug loading performance by modifying the surface morphology of DCBs at present. For example, an ultraviolet laser may be used to grind an outer surface of a balloon to create a nonplanar structure having concavities and convexities, which can lead to an increased specific surface area and strengthened adhesion of the balloon surface to a drug coating. Although this approach can significantly increase the drug adsorption and storage capacities of balloon surface and contact area of balloon surface with tissue of a blood vessel, it may degrade the balloon's properties, especially physical properties, such as a lowered rated burst pressure (RBP). In addition, it is also difficult to control the machining accuracy in order to avoid one or more strength weakening portions of the balloon created by overlaps of grinding areas.

Besides, the approach of plasma activation process is also used to introduce hydrophilic groups on balloon surface, which can also increase adhesion of drug coating to the balloon surface for facilitating the load of drugs on balloon surface. For example, some existing approaches utilize the plasma activation technique to introduce hydrophilic groups onto a balloon surface, which can form hydrogen bonds with active drugs contained in a drug layer. Although such hydrogen bonds can result in enhanced adhesion of the drug layer to the balloon surface and thus allow a higher drug load on the balloon surface, they are saturable, directional and specific to the charge property of the selected drug. In this way, this approach has a limited impact on adhesion of the drug coating.

Further, the approach of wrapping a layer of drug release film having elongated sealing slits and pores around the balloon surface can also be used, so as to protect the drug from being washed away by blood flow during delivery and expose and transfer the substantial drug into the blood vessel to achieve therapeutic purpose when the sealing slits and pores are opened in the dilation of balloon body. The prior art provides the method of wrapping a layer of drug release film having elongated sealing slits and pores around the balloon surface, in which the outer drug release film is attached to the straight smooth section of inner balloon by glue or laser welding to create a layer of drug storage pouch that reduces drug loss during delivery. However, such protective film has a thickness of about 0.01mm-0.1mm, which greatly affect the outer diameter through which the drug balloon passes. Moreover, such balloon is complicated in structure, difficult to fabricate and associated with a very high risk because it cannot be guaranteed that the film will not be damaged or stripped off and remain in the patient's body during the procedure.

## SUMMARY

An object of the present application is to provide a medical tube and method of preparing the medical tube, which can solve the above-described problems of existing drug-coated balloons, i.e., easy stripping off of a drug coating from the balloon surface during delivery and withdrawal, a non-uniform cover of the drug on the balloon surface and a low drug release rate and drug release speed during release.

To solve the above problems, present application provides the method of preparing a medical tube, comprising the following steps of: providing an interventional carrier and an etching solution; and immersing the interventional carrier in the etching solution for etching to obtain a medical tube with a porous surface.

Additionally, prior to the immersion of the interventional carrier in the etching solution, the method further comprises, filling the interventional carrier with a fluid for pressurization to make the interventional carrier dilate.

Additionally, the dilated interventional carrier is ultrasonically cleaned with a cleaning liquid and then dried.

Additionally, the etching solution comprises a mixed solution of an etchant and a diluent, and wherein the etchant is selected from an acidic solution, and the diluent is selected from alcohols and/or water.

Preferably, the acidic solution is selected from the group consisting of concentrated sulfuric acid, concentrated nitric acid, formic acid, acetic acid and mixtures thereof.

Preferably, the diluent is selected from the group consisting of glycerol, ethanol, glycol, water and mixtures thereof.

Preferably, a volume ratio of the etchant to the diluent ranges from 1:0.01 to 1:100.

Preferably, the interventional carrier is immersed in the etching solution to etch for a duration of time ranging from 1 second to 30 minutes.

Preferably, the interventional carrier is made of a material selected from the group consisting of polyether, segmented copolymer containing polyether , polyamide, segmented copolymer containing polyamide, polylactic acid, segmented copolymer containing polylactic acid, polyglycolic acid, copolymer of lactic acid and glycolic acid, segmented copolymer containing polyglycolic acid, polycaprolactone, segmented copolymer containing polycaprolactone, polyhydroxybutyrate, segmented copolymer containing polyhydroxybutyrate, hydrolyzable polyester and combinations thereof.

The present application also provides a medical tube comprising a substrate layer and a porous layer on a surface of the substrate layer.

Additionally, the porous layer is located at an inner or outer surface of the medical tube and comprises a plurality of pores with a diameter ranging from 0.1$\mu$m to 1000 $\mu$m.

Preferably, the medical tube is a drug-eluting balloon and a drug coating layer is loaded on the porous layer.

Additionally, a plurality of the porous layers is formed at the outer surface of the medical tube and a part of pores on different porous layers interconnects to define a hollow-out structure.

Additionally, the medical tube is made of a polymer material selected from the group consisting of polyether, segmented copolymer containing polyether , polyamide, segmented copolymer containing polyamide, polylactic acid, segmented copolymer containing polylactic acid, polyglycolic acid, copolymer of lactic acid and glycolic acid, segmented copolymer containing polyglycolic acid, polycaprolactone, segmented copolymer containing polycaprolactone, polyhydroxybutyrate, segmented copolymer containing polyhydroxybutyrate, hydrolyzable polyester and combinations thereof..

In summary, the medical tube and preparation method of present application have the following advantages over the prior art:

1) Improved Drug Loading Performance

The surface of the medical tube has a porous structure with uniformly distributed micron-scale pores, thereby allowing effective storage of a large amount of a drug into micropores and minimizing drug loss of the drug layer during delivery of the balloon toward a target lesion site as well as withdrawal therefrom after expansion and release of the balloon. That is, the significant stripping off of drug particles from the carrier surface under the scour of blood flow is able to be relieved.

2) Increased Drug Release Efficiency

The specific surface area of medical tube is significantly increased. That is, when the balloon is dilated after arriving at lesion site, the contact area of present carrier with the wall of the blood vessel is remarkably larger than that of common carrier surfaces. As a result, the drug release efficiency is able to be significantly increased.

3) Reduced Spraying Amount of Drug and Saved Cost

Due to presence of the micropores, the surface of the medical tube exhibits significantly enhanced adhesion to a drug coating, which enables to achieve more enhanced drug absorption efficient in the drug spray process. As a result, a desired drug load on the balloon surface is able to be achieved with a reduced drug amount sprayed onto the surface, resulting in effective cost savings.

4) Maintained Overall Strength of Interventional Carrier

The preparation method of medical tube in present application employs a mild surface treatment process that is easy to be controlled accurately and does not involve mechanical grinding. Thus, overall degradation of physical properties is able to be mitigated, and the problem of a reduced rated burst pressure (RBP) caused by groove engraving in the conventional balloons is able to be overcome.

5) Simplified Preparation Process

The preparation method of medical tube in present application is simple and easy to do with a low cost. Since only involving immersion and etching in a prepared acidic solution, the preparation process is simple and efficient without any large-scale equipment. Moreover, additional material such as a drug release film, is able to be avoided, so as to remove the risk that the additional material is stripped off and remain within a patient's body.

## BRIEF DESCRIPTION OF THE DRAWINGS

Fig. 1 is a structural schematic of a medical tube according to an embodiment of the present application.

Fig. 2 is a scanning electron microscope (SEM) image at 1000x magnification showing a surface of a part of a medical tube according to an embodiment of the present application.

Fig. 3 is a flowchart schematically illustrating the method of preparing a medical tube according to an embodiment of the present application.

Fig. 4 is a flowchart schematically illustrating a step of pre-treating an interventional carrier in a method of preparing a medical tube according to an embodiment of the present application.

Fig. 5 is a structural schematic of a tube with a porous layer according to an embodiment of the present application.

Fig. 6 shows SEM images at 1000x magnification of surfaces of parts of medical tubes according to a set of embodiments of the present application.

Fig. 7 shows SEM images at 1000x magnification of surfaces of parts of medical tubes according to a set of embodiments of the present application.

## DETAILED DESCRIPTION

The medical tube and preparation method provided in the present application will be described in greater detail below by way of particular embodiments with reference to the accompanying drawings. Advantages and features of the present application will be more apparent from the following description and appended claims. Note that the figures are provided in a very simplified form with the only intention to facilitate convenience and clarity in explaining embodiments of present application.

The medical tube described in the present application may be either a medical balloon or an interventional or non-interventional catheter. Particular examples of it may include drug-coated balloons, tracheal ventilation tubes, drainage tubes, venous catheters, micro-catheters, infusion tubes, nasal oxygen cannulas and the like suitable for use in humans or other mammals.

The solution of present application will be described below with the medical balloon as an example, but the following embodiments are also applicable to any of the aforementioned various types of tubes.

After plasma etching process, a porous structure having nanoscale pores of 100-500 nm is formed on the surface of a drug-coated balloon. When viewed with naked eye, the balloon surface with such structure still appears to be smooth and transparent. And, such nanoscale porous structure does not have a remarkable effect on specific surface area of conventional balloons, thereby failing to dramatically raise adhesion of the balloon surface to a drug coating

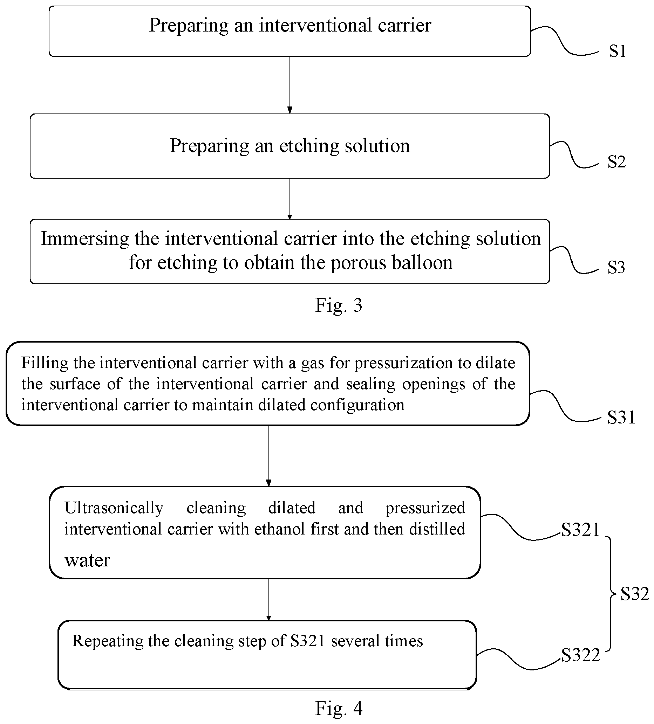

Embodiments of the present application provide a method for preparing a medical tube, which etches the balloon by immersing in an etching solution. As shown in Fig. 3, the method includes the following steps. In S1, preparing an interventional carrier is prepared. The specific preparation methods of interventional carrier made of polymer material are not limited in present application and any suitable method known in the art can be used. In addition, in other embodiments of the present application, the interventional carrier may be commercially available and obtained by purchase. In step S2, an etching solution is prepared. In step S3, the interventional carrier is immersed in the etching solution for etching, thereby obtaining a medical tube with a porous surface, which is a porous balloon here. Steps S1 and S2 may be performed either simultaneously or successively. That is, the Step S1 of preparing the interventional carrier can follow step S2 of preparing the etching solution. In embodiments of the present application, the interventional carrier may be formed of a material selected from the group consisting of polyether, segmented copolymer containing polyether , polyamide, segmented copolymer containing polyamide, polylactic acid, segmented copolymer containing polylactic acid, polyglycolic acid, copolymer of lactic acid and glycolic acid, segmented copolymer containing polyglycolic acid, polycaprolactone, segmented copolymer containing polycaprolactone, polyhydroxybutyrate, segmented copolymer containing polyhydroxybutyrate, hydrolyzable polyester and combinations thereof.

Compared with the conventional balloons, the porous balloon obtained from the preparation method provided in embodiments of present application has a porous surface with micron-scale pores that are identifiable to the naked eye (i.e., the surface has a frosted texture). This micron-scale porous surface is more favorable to drug storage and release and allows enhanced adhesion of a drug coating to the porous balloon, thus enabling to solve the above problems of existing balloons, i.e., easy stripping off of a drug coating from the balloon surface during delivery and withdrawal, a non-uniform cover of the drug over the balloon surface and a low drug release rate and speed during drug release. Moreover, such porous surface will not degrade the balloon's physical properties, and avoids the problem of degraded balloon properties, especially physical properties, such as a reduced balloon rated burst pressure (RBP) caused by groove engraving.

Prior to the step 3 of immersing interventional carrier into etching solution for etching, in order to achieve a uniform etching of the interventional carrier, the above preparation method further includes pretreating the interventional carrier and etching the pretreated interventional carrier.

As shown in Fig. 4, the pre-treatment may include the following steps. In step S31, the interventional carrier is filled with a fluid for pressurization (in this embodiment, a gas is filled for pressurization) to dilate the surface of the interventional carrier and openings of the interventional carrier are sealed to maintain dilated configuration. In step S32, the dilated interventional carrier is ultrasonically cleaned with a cleaning liquid and then dried.

The cleaning liquid may be selected from ethanol and distilled water. The ultrasonic cleaning process may include the following steps. In step S321, the interventional carrier that has been filled with gas for pressurization is ultrasonically cleaned by ethanol first and then by distilled water. In step S322, the cleaning cycle described in step S321 is repeated for several times, e.g., 2-3 times. Of course, in other embodiments, cleaning liquid may be only ethanol. Alternatively, the interventional carrier that has been filled with gas for pressurization may be ultrasonically cleaned by distilled water first and then by ethanol, and the present application is not limited in the sequence.

The pre-treatment is performed to obtain a more uniform frosted surface in the subsequent etching step, that is, to obtain a more uniform distribution of pores.

The etching solution used in above step S2 may be prepared by mixing an etchant with a diluent. The etchant may be selected from various acidic solutions. For example, the etchant may be selected from one of concentrated sulfuric acid (98%), concentrated nitric acid (68%), formic acid and acetic acid, or the mixture of several acids. The diluent may be selected from various alcohols or water may be used as diluent. The alcohols may include, but are not limited to, glycerol, ethanol and glycol. Of course, the etchant may be composed of etching solution without diluent.

In particular, the volume ratio of the etchant to the diluent ranges from 1:0.01 to 1:100. It is to be noted that the selections to various etchants and diluents and the ratio between etchants and diluents would largely affect etching performance of the etching solution and hence affect the sizes of micropores on the surface of the etched polymer. As an example, for a balloon made of nylon 12, the etching solution consisting of concentrated sulfuric acid and glycerol has a gradually weakening etching performance with a volume ratio of the etchant to the diluent decreasing from 1:1 to 1:10, and does not possess an etching performance with a volume ratio dropping below 1:10; For the same balloon, the etching solution consisting of concentrated nitric acid and water has a gradually weakening etching performance with a volume ratio of the etchant to the diluent decreasing from 1:2 to 1:15, and does not possess an etching performance with a volume ratio dropping below 1:15; the etching solution consisting of formic acid and ethanol has a gradually weakening etching performance with a volume ratio of the etchant to the diluent decreasing from 1:2 to 1:15, and does not possess an etching performance with a volume ratio dropping below 1:15; the etching solution consisting of acetic acid and glycol has a gradually weakening etching performance with a volume ratio of the etchant to the diluent decreasing from 1:2 to 1:15, and does not possess an etching performance with a volume ratio dropping below 1:15; the etching solution consisting of acetic acid and glycerol has a gradually weakening etching performance with a volume ratio of the etchant to the diluent decreasing from 1:1.5 to 1:10, and does not possess an etching performance with a volume ratio dropping below 1:10.

Preferably, in order to achieve desired balloon surface morphology, different etching solutions having different etching performance requires different etching time for achieving required etching effect. Preferably, the interventional carrier is generally immersed in the etching solution for 1 second to 30 minutes.

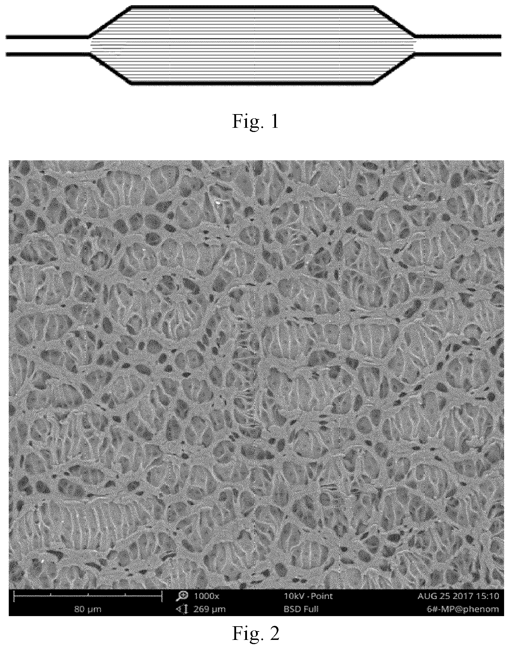

Embodiments of present application also provide a porous balloon with a network structure, and are intended to load a drug. The outer surface of the porous balloon has at least one porous layer in which a number of pores are formed. Preferably, the pores have a diameter in the range of 0.1-1000 microns. Unlike a regular smooth balloon surface or a balloon surface with nanoscale micropores, a surface of the porous balloon of present application is a porous surface with uniformly distributed micron-scale pores that are identifiable to naked eyes (i.e., a frosted texture surface) prior to the coating of drug. The frosted texture surface covers planar section and tapered section of the balloon (as shown in Fig. 1). Preferably, the frosted texture surface has a porous structure with various diameters in the range of 0.1-1000 microns. The size range of the micropores may be effectively controlled by properly setting operational parameters of the above preparation method. Those skilled in the art will appreciate that, although the size range of pores has been described as being in the range of 0.1-1000 microns in the above embodiments, it does not mean that the size of pores in the surface of the balloon obtained from the above method has to be within the range, because practical pore size measurement depends on a resolution of the measuring method used. All smaller pores, e.g., with a diameter in the range of 0.1-1 micron may be effectively measured by a high-resolution measurement method, and smaller pores with a diameter lower than 1 micron may not be identified in a low-resolution measurement method with a detection limit above 1 micron. Therefore, according to the present application, pores in the balloon are not limited to having a diameter within the aforementioned range of 0.1-1000 microns, and any balloon obtained from the above preparation method is considered to fall within the scope of the present application.

According to the present application, pores in the porous layer of the porous balloon may be uniformly distributed. Such a uniform distribution enables to ensure a uniform distribution of drug on balloon surface when loading a drug coating layer. Of course, the present application is not so limited, because the pores may alternatively have a non-uniform distribution and/or size.

In addition, when a plurality of porous layers are formed in the porous balloon, all or some of pores communicate with each other to form a hollow-out structure. Generally, the porous balloon with a hollow-out structure has a porosity greater than 50%, as shown in Fig. 2 that shows a scanning electron microscope (SEM) image at 1000x magnification of a surface of a part of the porous balloon. The hollow-out structure can additionally increase a contact area of the balloon surface with blood, making the balloon more favorable to drug storage and release.

According to the present application, the porous layer of the porous balloon may be made of a polymer material selected from the group consisting of polyether, segmented copolymer containing polyether , polyamide, segmented copolymer containing polyamide, polylactic acid, segmented copolymer containing polylactic acid, polyglycolic acid, copolymer of lactic acid and glycolic acid, segmented copolymer containing polyglycolic acid, polycaprolactone, segmented copolymer containing polycaprolactone, polyhydroxybutyrate, segmented copolymer containing polyhydroxybutyrate, hydrolyzable polyester and combinations thereof. The porous layer may be in the form of a monolayer or a multilayer.

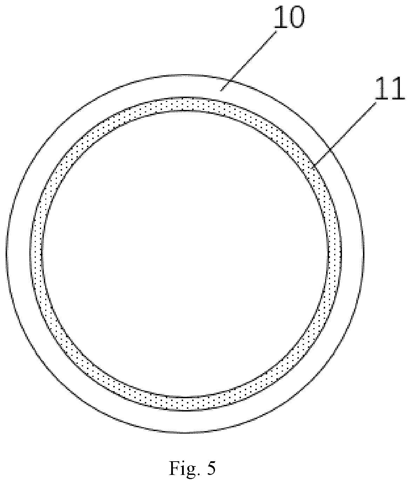

In an embodiment, the porous layer is located at an inner surface of the medical tube. As shown in Fig. 5, an infusion tube includes a substrate layer 10 and a porous layer 11 on an inner surface of the substrate layer. The porous layer 11 may include a network structure and its surface morphology may be as shown in the SEM image of Figs. 2, 6 or 7. In use, a drug may be loaded in the inner surface of the infusion tube and flow into a patient's body together with a fluid flowing in the infusion tube.

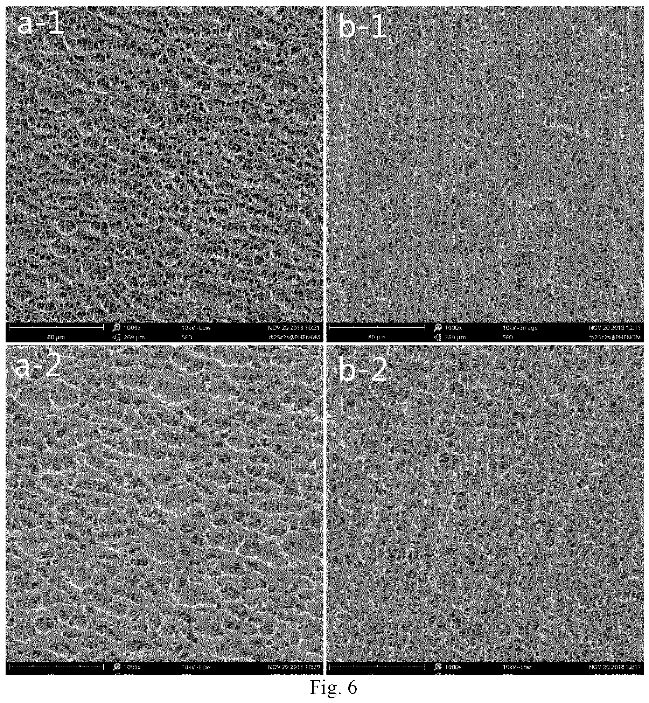

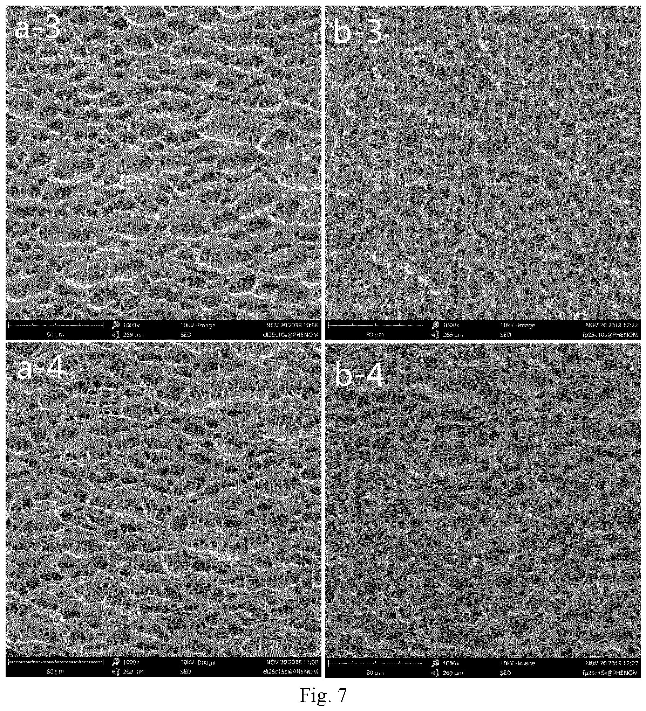

Figs. 6 and 7 show SEM images at 1000x magnification of surfaces of parts of medical tubes, where samples a and b are SEM images etched at different etching conditions. Sample a is made of a polyether-amide segmented copolymer with a shore hardness of 55D, and sample b is made of a polyether-amide segmented copolymer with a shore hardness of 72D. The images numbered 1-4 illustrate samples etched for different etching time with same etchant, where the etching time gradually increases from number 1 to 4. As seen from Figs. 6 and 7, the porous layers with network structures are able to be formed on surfaces of medical tubes made of different materials under different etching durations.

The preparation method of present application will be described below in even greater detail with reference to particular examples in order to further demonstrate features and advantages of the present application.

Example 1

In this example, the method includes the following steps, with the concentrated sulfuric acid (98%) selected as etchant and the purified water selected as diluent.

1.1: Pre-treatment of samples: An interventional carrier was connected to an inflator to fill with gas for pressurization so that a surface of the carrier was completely dilated, and the openings of interventional carrier were then closed so as to be maintained the dilated configuration. The dilated carrier was ultrasonically cleaned in ethanol and then in distilled water. This cleaning cycle was repeated for 2-3 times, followed by drying of the carrier.

1.2: Preparation of etching solution. Various glass containers were cleaned, and concentrated sulfuric acid (98%, etchant) and purified water (diluent) were measured with graduated cylinders and mixed at a volume ratio of 1:1. The mixture was stirred gently with a glass rod, poured into a clean airtight bottle and cooled to room temperature.

1.3: Etching Treatment. The pre-treated interventional carrier was completely immersed in the prepared etching solution for 20 seconds and then taken out therefrom. The carrier was immediately cleaned with purified water, and water droplets remaining on the surface thereof were wiped off, followed by natural drying. In this way, the porous balloon according to present application was obtained.

Example 2

In this example, three porous balloons (each balloon with a pore size measured to be within the range of 1-30 microns through pore size tests) prepared in accordance with Example 1 and three regular balloons (each regular balloon having a smooth surface without pores) that have not been etched were compared for their drug release performance, and the results are summarized in Table 1. A number of metrics used in performance assessment are defined as follows:

$$\text{Proportion of Drug Particles Released during Delivery} = \text{Number of Drug Particles Released during Delivery} / (\text{Number of Drug Particles Released during Delivery} + \text{Number of Drug Particles Released during Expansion and Retraction} + \text{Number of Drug Particles Released during Withdrawal})$$

$$\text{Proportion of Drug Particles Released during Expansion and Retraction} = \text{Number of Drug Particles Released during Expansion and Retraction} / (\text{Number of Drug Particles Released during Delivery} + \text{Number of Drug Particles Released during Expansion and Retraction} + \text{Number of Drug Particles Released during Withdrawal})$$

$$\text{Proportion of Drug Particles Released during Withdrawal} = \text{Number of Drug Particles Released during Withdrawal} / (\text{Number of Drug Particles Released during Delivery} + \text{Number of Drug Particles Released during Expansion and Retraction} + \text{Number of Drug Particles Released during Withdrawal})$$

$$\text{Drug Release Rate} = (\text{Theoretical Amount of Drug Coated on Balloon Surface} - \text{Residual Amount of Drug on Balloon Surface after Test}) / \text{Theoretical Amount of Drug Coated on Balloon Surface}$$

Table 1 Drug Release Performance Comparison between Porous Balloon and non-etched Regular Balloons

| Sample | Steps | Proportions of Drug Particles Released | Drug Release Rate |
|---|---|---|---|
| Regular Balloon 1 | Delivery | 38.94% | 68% |
| | Dilation and Retraction | 35.60% | |
| | Withdrawal | 25.45% | |
| Regular Balloon 2 | Delivery | 49.74% | 64% |
| | Dilation and Retraction | 28.34% | |
| | Withdrawal | 21.92% | |
| Regular Balloon 3 | Delivery | 29.00% | 69% |
| | Dilation and Retraction | 40.63% | |
| | Withdrawal | 30.37% | |
| Porous Balloon 1 | Delivery | 6.85% | 85% |
| | Dilation and Retraction | 61.58% | |
| | Withdrawal | 16.95% | |
| Porous Balloon 2 | Delivery | 13.81% | 86% |
| | Dilation and Retraction | 53.75% | |
| | Withdrawal | 18.40% | |
| Porous Balloon 3 | Delivery | 18.16% | 72% |
| | Dilation and Retraction | 63.45% | |
| | Withdrawal | 18.38% | |

As can be seen from Table 1, the porous balloons having frosted microporous surfaces according to present application have better drug loading performances. This is because micron-scale pores present in the surface of each interventional carrier allow effective storage of a large drug amount into the micron-scale pores and minimizing drug loss of the drug layer during delivery of the balloon toward a target lesion site as well as withdrawal therefrom after expansion and release of the balloon. That is, the significant stripping off of drug particles from the carrier surface under the scour of blood flow is able to be relieved. As seen from the data in Table 1, compared with the regular balloons, each of the three porous balloons exhibits a significant reduction in the proportions of drug particles released during delivery and withdrawal.

Moreover, the porous balloons having frosted microporous surfaces according to present application also exhibit higher drug release rates during drug release. Due to the uniformly distributed micropores, the surface of each interventional carrier has an enhanced specific surface area. After the interventional carrier is delivered to the lesion site and is dilated, their surface has a larger contact area with the wall of the blood vessel than the surface of regular balloons, which results in a significant increase in drug release rate. The data in Table 1 show that, compared with the regular balloons, each of the three porous balloons exhibits a significant increase in both the proportion of drug particles released during dilation and retraction and the drug release rate.

In summary, the medical tube and preparation method of present application have the following advantages over the prior art:

1) Improved Drug Loading Performance

The surface of the medical tube has a porous structure with uniformly distributed micron-scale pores, thereby allowing effective storage of a large amount of a drug into micropores and minimizing drug loss of the drug layer during delivery of the balloon toward a target lesion site as well as withdrawal therefrom after expansion and release of the balloon. That is, the significant stripping off of drug particles from the carrier surface under the scour of blood flow is able to be relieved.

2) Increased Drug Release Efficiency

The specific surface area of medical tube is significantly increased. That is, when the balloon is dilated after arriving at lesion site, the contact area of present carrier with the wall of the blood vessel is remarkably larger than that of common carrier surfaces. As a result, the drug release efficiency is able to be significantly increased.

3) Reduced Spraying Amount of Drug and Saved Cost

Due to presence of the micropores, the surface of the medical tube exhibits significantly enhanced adhesion to a drug coating, which enables to achieve more enhanced drug absorption efficient in the drug spray process. As a result, a desired drug load on the balloon surface is able to be achieved with a reduced drug amount sprayed onto the surface, resulting in effective cost savings.

4) Maintained Overall Strength of Interventional Carrier

The preparation method of medical tube in present application employs a mild surface treatment process that is easy to be controlled accurately and does not involve mechanical grinding. Thus, overall degradation of physical properties is able to be mitigated, and the problem of a reduced rated burst pressure (RBP) caused by groove engraving in the conventional balloons is able to be overcome.

5) Simplified Preparation Process

The preparation method of medical tube in present application is simple and easy to do with a low cost. Since only involving immersion and etching in a prepared acidic solution, the preparation process is simple and efficient without any large-scale equipment. Moreover, additional material such as a drug release film, is able to be avoided, so as to remove the risk that the additional material is stripped off and remain within a patient's body.

The description presented above is merely that of a few preferred embodiments of the present application and does not limit the scope thereof in any sense. Any and all changes and modifications made by those of ordinary skill in the art based on the above disclosure fall within the scope as defined in the appended claims.

**Claims**

1. A method of preparing a medical tube, comprising the steps of:

   providing an interventional carrier and an etching solution; and
   immersing the interventional carrier in the etching solution for etching to obtain a medical tube with a porous surface.

2. The method according to claim 1, further comprising, prior to the immerse of the interventional carrier in the etching solution,
   filling the interventional carrier with a fluid for pressurization to make the interventional carrier dilate.

3. The method according to claim 2, wherein the dilated interventional carrier is ultrasonically cleaned with a cleaning liquid and then dried.

4. The method according to claim 1, wherein the etching solution comprises a mixed solution of an etchant and a diluent, and wherein the etchant is selected from an acidic solution, and the diluent is selected from alcohols and/or water.

5. The method according to claim 4, wherein the acidic solution is selected from the group consisting of concentrated sulfuric acid, concentrated nitric acid, formic acid, acetic acid and mixtures thereof.

6. The method according to claim 4, wherein the diluent is selected from the group consisting of glycerol, ethanol, glycol, water and mixtures thereof.

7. The method according to claim 4, wherein a volume ratio of the etchant to the diluent ranges from 1:0.01 to 1:100.

8. The method according to claim 1, wherein the interventional carrier is immersed in the etching solution to etch for a duration of time ranging from 1 second to 30 minutes.

9. The method according to claim 1, wherein the interventional carrier is made of a material selected from the group consisting of polyether, segmented copolymer containing polyether , polyamide, segmented copolymer containing polyamide, polylactic acid, segmented copolymer containing polylactic acid, polyglycolic acid, copolymer of lactic acid and glycolic acid, segmented copolymer containing polyglycolic acid, polycaprolactone, segmented copolymer containing polycaprolactone, polyhydroxybutyrate, segmented copolymer containing polyhydroxybutyrate, hydrolyzable polyester and combinations thereof.

10. A medical tube, comprising a substrate layer and a porous layer on a surface of the substrate layer.

11. The medical tube according to claim 10, wherein the porous layer is located at an inner or outer surface of the medical tube and comprises a plurality of pores with a diameter ranging from 0.1$\mu$m to 1000 $\mu$m.

12. The medical tube according to claim 10, wherein the medical tube is a drug-eluting balloon and a drug coating layer is loaded on the porous layer.

13. The medical tube according to claim 11, wherein a plurality of the porous layers is formed at the outer surface of the medical tube and a part of pores on different porous layers interconnects to define a hollow-out structure.

14. The medical tube according to claim 10, wherein the medical tube is made of a polymer material selected from the group consisting of polyether, segmented copolymer containing polyether , polyamide, segmented copolymer containing polyamide, polylactic acid, segmented copolymer containing polylactic acid, polyglycolic acid, copolymer of lactic acid and glycolic acid, segmented copolymer containing polyglycolic acid, polycaprolactone, segmented copolymer containing polycaprolactone, polyhydroxybutyrate, segmented copolymer containing polyhydroxybutyrate, hydrolyzable polyester and combinations thereof.

Fig. 1

Fig. 2

Fig. 3

Fig. 4

Fig. 5

Fig. 6

Fig. 7

### INTERNATIONAL SEARCH REPORT

International application No.

**PCT/CN2019/121319**

| A. CLASSIFICATION OF SUBJECT MATTER |
|---|
| A61M 25/10(2013.01)i；A61L 29/14(2006.01)i；A61L 29/16(2006.01)i |

According to International Patent Classification (IPC) or to both national classification and IPC

| B. FIELDS SEARCHED |
|---|
| Minimum documentation searched (classification system followed by classification symbols) |
| A61M25/-；A61L29/- |

Documentation searched other than minimum documentation to the extent that such documents are included in the fields searched

Electronic data base consulted during the international search (name of data base and, where practicable, search terms used)

CNPAT, CNKI, WPI, EPODOC: 微创医疗, 戴明欣, 吴晓蕾, 郭芳, 赵月根, 阚亦云, 管材, 球囊, 药, 微孔, 聚合物, 孔, 酸, 腐蚀, 刻蚀, 浸泡, balloon, etch, microporous, apertures, porous, hole, tube, pipe, catheter, canal, acid+

| C. DOCUMENTS CONSIDERED TO BE RELEVANT |

| Category* | Citation of document, with indication, where appropriate, of the relevant passages | Relevant to claim No. |
|---|---|---|
| X | CN 101199873 A (LEPU MEDICAL TECHNOLOGY (BEIJING) CO., LTD.) 18 June 2008 (2008-06-18) <br> description, page 2, line 6 to page 8, line 20, and figures 4-11 | 1-14 |
| X | CN 1919353 A (DONG, Heyan et al.) 28 February 2007 (2007-02-28) <br> description, page 2, line 6 to page 4, line 17 | 1, 4-8, 10-11 |
| X | CN 101869723 A (ZHAO, Jing) 27 October 2010 (2010-10-27) <br> description, paragraphs [0007]-[0021], and figure 1 | 1, 4-8, 10-11 |
| A | CN 103948972 A (BROSMED MEDICAL CO., LTD.) 30 July 2014 (2014-07-30) <br> entire document | 1-14 |
| A | US 6585926 B1 (ADVANCED CARDIOVASCULAR SYSTEMS, INC.) 01 July 2003 (2003-07-01) <br> entire document | 1-14 |

☐ Further documents are listed in the continuation of Box C.  ☑ See patent family annex.

| * Special categories of cited documents: | "T" later document published after the international filing date or priority date and not in conflict with the application but cited to understand the principle or theory underlying the invention |
|---|---|
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| **16 February 2020** | **24 February 2020** |

| Name and mailing address of the ISA/CN | Authorized officer |
|---|---|
| **China National Intellectual Property Administration** <br> **No. 6, Xitucheng Road, Jimenqiao Haidian District, Beijing 100088** <br> **China** | |
| Facsimile No. **(86-10)62019451** | Telephone No. |

Form PCT/ISA/210 (second sheet) (January 2015)

EP 3 888 733 A1

## INTERNATIONAL SEARCH REPORT
### Information on patent family members

International application No.

**PCT/CN2019/121319**

| Patent document cited in search report | | | Publication date (day/month/year) | Patent family member(s) | | | Publication date (day/month/year) |
|---|---|---|---|---|---|---|---|
| CN | 101199873 | A | 18 June 2008 | CN | 101199873 | B | 19 June 2013 |
| | | | | US | 2009112310 | A1 | 30 April 2009 |
| | | | | WO | 2008071047 | A1 | 19 June 2008 |
| CN | 1919353 | A | 28 February 2007 | CN | 100400113 | C | 09 July 2008 |
| CN | 101869723 | A | 27 October 2010 | None | | | |
| CN | 103948972 | A | 30 July 2014 | CN | 103948972 | B | 29 June 2016 |
| US | 6585926 | B1 | 01 July 2003 | None | | | |

Form PCT/ISA/210 (patent family annex) (January 2015)

17